# EUROPÄISCHE PATENTSCHRIFT

(11) **EP 1 230 892 B1**
(45) Veröffentlichungstag und Bekanntmachung des Hinweises auf die Patenterteilung: **14.06.2006**
(21) Anmeldenummer: 02090050.2
(22) Anmeldetag: 07.02.2002
(51) Int. Cl.: A61B 1/005, A61B 1/04, A61M 25/01

(54) **Endoskopischer Katheter**
Endoscopic catheter
Cathéter endoscopique

(30) Priorität: 10.02.2001 DE 10107586
(43) Veröffentlichungstag der Anmeldung: 14.08.2002
(73) Patentinhaber: BIOTRONIK GmbH & Co. KG, 12359 Berlin (DE)
(72) Erfinder: Müssig, Dirk, 90439 Nürnberg (DE); Schaldach, Max, verstorben (DE)
(74) Vertreter: Lindner-Vogt, Karin L.

(56) Entgegenhaltungen:
- EP-A- 1 072 281
- WO-A-94/10897
- WO-A-97/28839
- US-A- 4 786 155
- US-A- 5 254 088
- US-A- 6 079 414
- US-B1- 6 309 345

## Beschreibung

Die Erfindung betrifft einen Katheter, der zum Einführen in Körperhohlräume ausgebildet ist. Der Katheter umfasst einen distalen Katheterabschnitt, eine Beleuchtungseinrichtung, eine Bildaufnahmeeinheit sowie eine Bildwiedergabeeinheit. Die Beleuchtungseinrichtung ist ausgebildet, eine Umgebung des distalen Katheterabschnitts mit elektromagnetischer Strahlung zu beleuchten. Die Bildaufnahmeeinheit ist ausgebildet, ein Bild der von der Umgebung des distalen Katheterabschnitts reflektierten elektromagnetischer Strahlung aufzunehmen und zum distalen Ende des Katheters zu leiten. Die Bildwiedergabeeinheit ist mit dem proximalen Ende des Katheters verbunden und ausgebildet, ein Bild der aufgenommenen elektromagnetischen Strahlung wiederzugeben.

Derartige endoskopische Katheter sind beispielsweise aus der US-PS 6,110,106 und EP-A-1 072 281 hinlänglich bekannt und dienen der visuellen Untersuchung von Körperhohlräumen.

Ein Problem ergibt sich jedoch, wenn die zu untersuchenden Körperhohlräume gefüllt sind, so wie beispielsweise Blutgefäße mit Blut geführt sind.

Erfindungsgemäß besteht die Lösung dieses Problems in einem endoskopischen Katheter der eingangs genannten Art, der steuerbar zum Einführen in Körpergefäße, insbesondere Blutgefäße, ausgebildet ist, sowie dazu, ein Bild von der Umgebung des distalen Katheterabschnitts reflektierten elektromagnetischen Strahlung mit einer Wellenlänge wiederzugeben, für die das Blut eine hohe Transparenz besitzt. Ein derartiger Katheter erlaubt es in vorteilhafter Weise, auch blutgefüllte Gefäße endoskopisch zu untersuchen. Dies erlaubt es insbesondere, den Katheter optisch an einen bestimmten Ort zu steuern, an dem beispielsweise eine Gefäßdilatation vorgenommen werden soll, ein Stent gesetzt, oder Körpergewebe elektrisch stimuliert werden soll.

Vorzugsweise ist der Katheter ausgebildet, ein Bild in einem Wellenlängenbereich von 600 bis 650 Nanometern wiederzugeben. Dazu ist insbesondere die Beleuchtungseinrichtung ausgebildet, die Umgebung des distalen Katheterabschnitts mit Licht einer Wellenlänge von 600 bis 650 Nanometern zu beleuchten. Die Beleuchtungseinrichtung ist dazu vorzugsweise mit Bandpassfilter für ein Frequenzband von 600 bis 650 Nanometern ausgestattet. Außerdem umfasst die Beleuchtungseinrichtung vorzugsweise einen Lichtwellenleiter vom proximalen zum distalen Katheterende, der ausgebildet ist, zum Beleuchten dienende elektromagnetische Strahlung vom proximalen zum distalen Katheterende zu leiten. Wenn der Bandpassfilter in vorteilhafter Weise am proximalen Katheterende angeordnet ist, braucht durch den Lichtwellenleiter nur die zur Beleuchtung benötigte elektromagnetische Strahlung geleitet zu werden und der Lichtwellenleiter kann an deren Wellenlänge optimal angepasst werden.

Vorzugsweise ist der endoskopische Katheter als Elektrodenleitung ausgebildet und ist dazu an seinem distalen Katheterabschnitt mit mindestens einer Elektrode zur Abgabe und/oder Aufnahme elektrischer Signale an oder von an den distalen Katheterabschnitt angrenzendes Körpergewebe versehen. Ein derartiger, beispielsweise mit Stimulationselektroden versehener Katheter kann in vorteilhafter Weise optisch zu seinem Bestimmungsort gesteuert werden.

Der distale Katheterabschnitt ist mit einem expandierbaren Ballon versehen. Dadurch ist der expandierbare Ballon entweder zum Dilatieren verengter Körpergefäße geeignet oder zum Einführen und Weiten von Stents ausgebildet. Mit einem derartigen Katheter kann ein Gefäß unter optischer Kontrolle geweitet werden oder ein Stent gesetzt und expandiert werden. Bei der letztgenannten Variante kann der gesetzte und expandierte Stent auf einfache Weise optisch kontrolliert werden.

Vorzugsweise ist der Katheter mit an sich bekannten Steuermitteln zum gezielten Auslenken des distalen Katheterendes ausgestattet. Dies erlaubt eine Steuerung des Katheters auf an sich bekannte Art und Weise und bietet gleichzeitig den Vorteil der endoskopischen Kontrolle der Kathetersteuerung.

Die Erfindung soll nun anhand eines Ausführungsbeispiels mit Hilfe der Figuren näher erläutert werden. Diese zeigen:
- Figur 1: einen Überblick über einen erfindungsgemäßen endoskopischen Katheter;

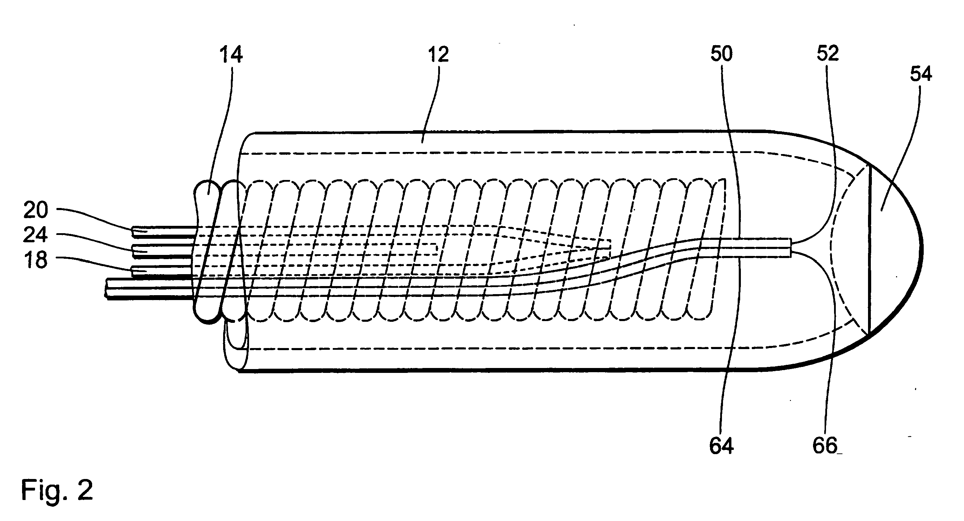

- Figur 2: das distale Ende des endoskopischen Katheters aus Figur 1 in vergrößerter Darstellung;
- Figur 3: das proximale Ende des endoskopischen Katheters mit daran angeschlossener Beleuchtungs- und Bildwiedergabeeinheit;
- Figur 4: beispielhaft einen Querschnitt durch eine Lichtleitfaser, wie sie in Figur 3 dargestellt ist.

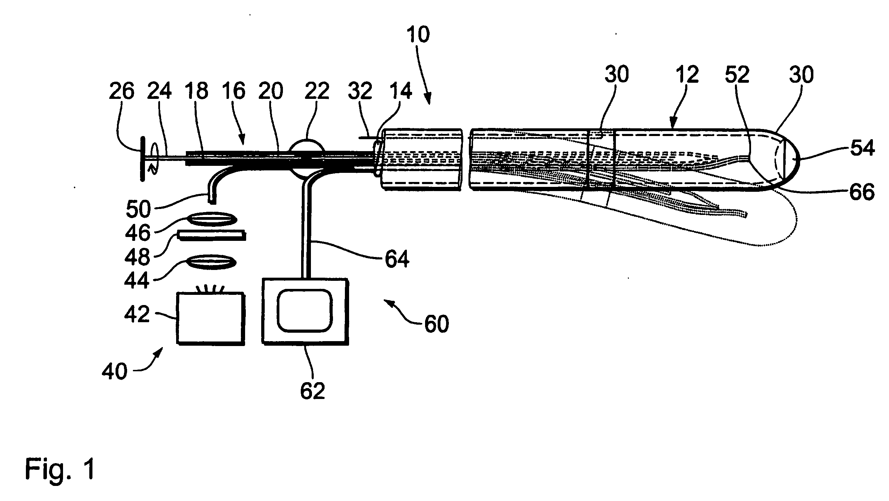

Der endoskopische Katheter 10 aus Figur 1 umfasst im wesentlichen drei Funktionsgruppen: die erste Funktionsgruppe umfasst einen steuerbaren, zum Einführen in Blutgefäße geeigneten Katheter mit einer Hülle 12, die eine versteifende, biegsame Heligswendel 14 einschließt, die in ihrem Inneren ein Lumen zur Aufnahme von Steuermitteln 16 umfasst, zu denen zwei Steuerdrähte 18 und 20 zählen, die an ihrem distalen Ende miteinander verbunden sind und im Bereich ihrem proximalen Endes über ein Handrad 22 relativ zueinander längst verschiebbar sind. Der Katheter 10 ist im Bereich seines distalen Endes flexibler ausgeführt, als in den daran anschließenden Bereichen bishin zum proximalen Ende. Durch Drehen an dem Handrad 22 und damit durch Längsverschieben der beiden Steuerdrähte 18 und 20 relativ zueinander kann das distale Ende des Katheters, wie in Figur 1 gestrichelt dargestellt, gezielt seitlich ausgelenkt oder deflektiert werden.

Zwischen den beiden Steuerdrähten 18 und 20 ist ein im wesentlichen torsionssteifes Flachband 24 vorgesehen, welches zumindest im Bereich des distalen Endes des Katheters 10 in Richtung der beiden Steuerdrähte 18 und 20 seitlich auslenkbar ist. Das Flachband 24 ist mit einem Griffstück 26 verbunden, mit welchen das Flachband 24 um seine Längsachse gedreht werden kann. Mit Drehen des Flachbandes 24 werden zumindest auch die beiden Steuerdrähte 18 und 20 rotiert, sodass mittels des Griffstückes 26 und des Flachbandes 24 die radiale Richtung einer Auslenkung des Katheterendes mittels der beiden Steuerdrähte 18 und 20 bestimmt werden kann.

Eine weitere Funktionseinheit wird bei dem in Figur 1 dargestellten Katheter von einer Stimulations- oder Sensing-Elektrode 30 im Bereich des distalen Endes des Katheters 10 gebildet, die über eine elektrische Leitung 32 mit dem proximalen Ende des Katheters 10 in elektrischer Verbindung steht. Anstelle der einen Elektrode 30 können auch mehrere Elektroden und entsprechend mehrere elektrische Leitungen vorgesehen sein. Ein derartiger Katheter kann als Stimulationselektrodenleitung beispielsweise im Zusammenhang mit einem Herzschrittmacher oder einem Defibrilator Verwendung finden.

In einer nicht dargestellten, alternativen Ausführungsvariante kann der Katheter 10 auch ein Ballonkatheter sein, der an seinem distalen Ende einen expandierbaren Ballon trägt, der beispielsweise zum Weiten von Gefäßen oder zum Einbringen von Stents in verengte Blutgefäße geeignet ausgebildet ist.

Eine dritte Funktionsgruppe dient der endoskopartigen Bildaufnahme der Umgebung des distalen Endes des Katheters 10. Diese dritte Funktionsgruppe umfasst eine Beleuchtungseinheit 40 mit einer Lichtquelle 42, einem Linsensystem 44 und 46, einem optischen Bandpassfilter 48 und einem Beleuchtungslichtwellenleiter 50. Die Beleuchtungseinheit 40 ist dabei im Bereich des proximalen Endes des Katheters 10 angeordnet und der Beleuchtungslichtwellenleiter 50 erstreckt sich vom proximalen Ende des Katheters 10 bis in die Nähe der Katheterspitze am distalen Ende des Katheters 10. Die Beleuchtungseinheit 40 ist so ausgelegt, dass zur Beleuchtung dienende elektromagnetische Strahlung im Wellenlängenbereich zwischen 600 und 650 Nanometern zum distalen Ende des Katheters 10 geleitet wird, und dort austreten kann, um die Umgebung der Katheterspitze zu beleuchten. Die Strahlung im Wellenlängenbereich zwischen 600 und 650 Nanometern tritt dabei an einem distalen Ende 52 des Beleuchtungslichtwellenleiters 50 aus und durch eine optische Linse 54 am distalen Ende des Katheters 10 hindurch. Die Linse 54 sowie die relative Position des distalen Beleuchtungslichtwellenleiters 52 zum Fokus der Linse 54 sind so gewählt, dass das aus dem Beleuchtungslichtwellenleiter 50 austretende infrarote Licht so verteilt wird, dass die Umgebung des distalen Endes des Katheters 10 gleichmäßig ausgeleuchtet wird. Die Wellenlänge der elektromagnetischen Strahlung bzw. des Infrarotlichtes im Bereich zwischen 600 und 650 Nanometern ist so gewählt, dass sie in einem Bereich liegt, in dem Blut im Wesentlichen transparent ist. Auf diese Weise kann die beleuchtende Strahlung durch in Blutgefäßen allenthaltend vorhandenes Blut hindurchtreten und wird erst von den Wänden der Blutgefäße reflektiert.

Zu der dritten Funktionseinheit zählt auch eine endoskopische Bildaufnahme- und Wiedergabeeinheit 60, die ein Bildwiedergabegerät 62 umfasst, welches über einen Bildlichtwellenleiter 64 mit dem distalen Ende des Katheters 10 verbunden ist. Der Bildlichtwellenleiter 64 endet im Fokus der Linse 54 und ist so ausgebildet, dass er ein auf eine distale Endfläche 66 des Bildlichtwellenleiters 64 projiziertes Bild der Umgebung des distalen Katheterendes aufnehmen und an seinem proximalen Ende wiedergeben kann. Das proximale Ende des Bildlichtwellenleiters 64 ist dazu mit der Bildwiedergabeeinheit 62 verbunden. Diese umfasst einen CCD-Chip, auf den ein optisch zum proximalen Ende des Bildlichtwellenleiters 64 übertragenes Bild der Umgebung des distalen Katheterendes projiziert wird. Dieses Bild ist ein Infrarotbild im Wellenlängenbereich zwischen 600 und 650 Nanometern und wird elektronisch in ein sichtbares Bild umgesetzt, welches auf einem Bildschirm der Bildwiedergabeeinheit 62 dargestellt wird.

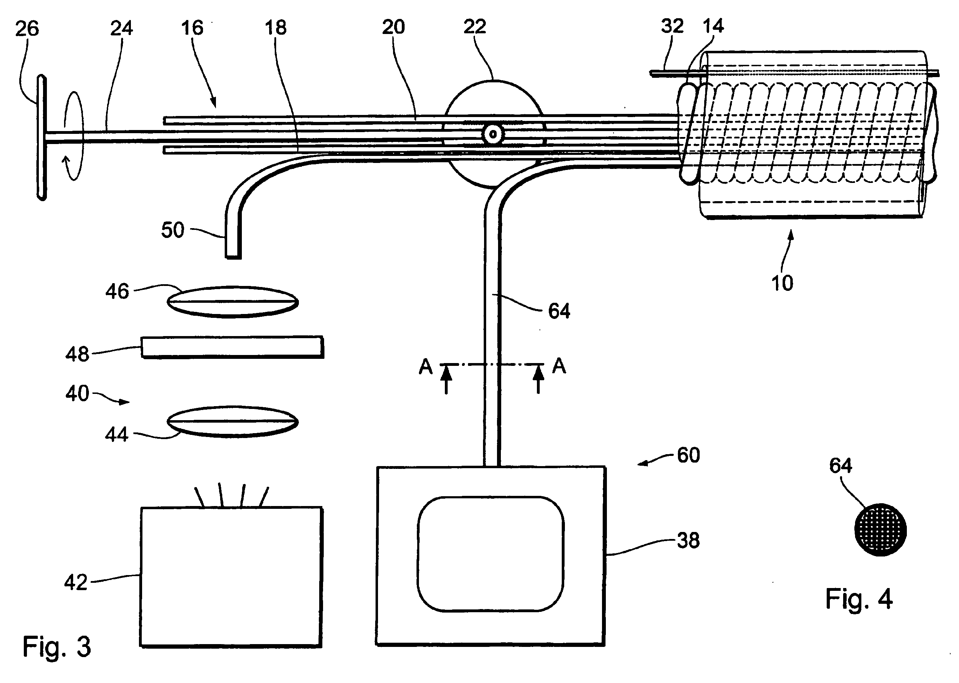

Figur 4 zeigt einen Querschnitt durch den Bildlichtwellenleiter 64, der viele einzelne lichtleitende Fasern enthält, von denen in Figur 4 der Übersicht halber nur wenige dargestellt sind. Jede einzelne Faser überträgt einen Bildpunkt des aufgenommenen Bildes.

Auf diese Weise kann ein Arzt beim Einführen des Katheters 10 auf dem Bildschirm der Bildwiedergabeeinheit 62 ständig das von der distalen Katheterspitze aufgenommene Bild der Gefäßwände betrachten, zwischen denen die Spitze des Katheters 10 gerade hindurchgeführt wird. Dies ermöglicht es dem Arzt in vorteilhafter Weise Gefäßverzweigungen zu erkennen, in die die distale Katheterspitze hineingesteuert werden soll. Auch kann der Arzt erkennen, ob sich die Katheterspitze bereits im Bereich einer zu behandelnden Gefäßverengung befindet, die mit einem in das distale Katheterende integrierten Ballon behandelt werden soll. Wenn das distale Katheterende wie in den Figuren abgebildet als Elektrodenleitung ausgebildet ist, kann diese Elektrodenleitung unter optischer Kontrolle genau an den vorgesehenen Orten platziert werden.

In einer Weiterbildung kann das distale Katheterende mehrere optische Systeme und Bildlichtwellenleiter umfassen, mit denen Bilder nicht nur über die Katheterspitze aufgenommen werden können, sondern auch an von der Katheterspitze etwas entfernten Orten des Katheters, beispielsweise im Bereich der Elektrode 30.

Ebenso können alternative Beleuchtungs- und Bildwiedergabeeinheiten verwendet werden, genauso wie die Verwendung alternativer Steuermittel möglich ist.

## Patentansprüche

1. Endoskopischer Katheter, ausgebildet zum Einführen in Körperhohlräumen, mit einem distalen Katheterabschnitt und einer Beleuchtungseinrichtung (40), die ausgebildet ist, eine Umgebung des distalen Katheterabschnitts mit elektromagnetischer Strahlung zu beleuchten sowie mit einer Bildaufnahmeeinheit (60), die ausgebildet ist, ein Bild der von der Umgebung des distalen Katheterabschnitts reflektierten elektromagnetischer Strahlung aufzunehmen und zum proximalen Ende des Katheters (10) zu leiten, sowie eine Bildwiedergabeeinheit (62), die mit dem proximalen Ende des Katheters (10) verbunden und ausgebildet ist, ein Bild der aufgenommenen elektromagnetischen Strahlung wiederzugeben, wobei der Katheter (10) steuerbar zum Einführen in Körpergefäße, insbesondere Blutgefäße, ausgebildet ist sowie dazu, ein Bild der von der Umgebung des distalen Katheterabschnitts reflektierten elektromagnetischen Strahlung mit einer Wellenlänge wiederzugeben, für die Blut eine hohe Transparenz besetzt,
**dadurch gekennzeichnet, dass** der Katheter (10) an seinem distalen Katheterabschnitt einen expandierbaren Ballon trägt, der zum Dilatieren verengter Körpergefäße geeignet ausgebildet ist.

2. Katheter nach Anspruch 1, **dadurch gekennzeichnet, dass** der Katheter (10) ausgebildet ist, ein in einem Wellenlängenbereich von 600 bis 650 Nanometern aufgenommenes Bild wiederzugeben.

3. Katheter nach Anspruch 2, **dadurch gekennzeichnet, dass** die Beleuchtungseinrichtung (40) ausgebildet ist, die Umgebung des distalen Katheterabschnitts mit infrarotem Licht einer Wellenlänge von 600 bis 650 Nanometern zu beleuchten.

4. Katheter nach Anspruch 3, **dadurch gekennzeichnet, dass** die Beleuchtungseinrichtung (40) einen optischen Bandpassfilter (48) für ein Frequenzband von 600 bis 650 Nanometern umfasst.

5. Katheter nach einem der Ansprüche 1 bis 4, **dadurch gekennzeichnet, dass** die Beleuchtungseinrichtung (40) einen Beleuchtungslichtwellenleiter (50) vom proximalen zum distalen Katheterende umfasst, der ausgebildet ist, zum Beleuchten dienende Elektromagnetische Strahlung vom proximalen zum distalen Katheterende zu leiten.

6. Katheter nach einem der vorhergehenden Ansprüche, **dadurch gekennzeichnet, dass** der Katheter (10) als Elektrodenleitung ausgebildet ist und dazu an seinem distalen Katheterabschnitt mit mindestens einer Elektrode zur Abgabe und/ oder Aufnahme elektrischer Signale an oder von an den distalen Katheterabschnitt angrenzendes Körpergewebe versehen ist.

7. Katheter nach Anspruch 1, **dadurch gekennzeichnet, dass** der Ballon zum Einführen und Weiten von Stents geeignet ausgebildet ist.

8. Katheter nach einem der vorhergehenden Ansprüche, **dadurch gekennzeichnet, dass** der Katheter (10) vom proximalen Ende des Katheters (10) betätigbare Steuermittel (16) zum gezielten Auslenken des distalen Katheter-endes umfasst.

## Claims

1. An endoscopic catheter, implemented for insertion into body cavities, having a distal catheter section and an illumination unit (40), which is implemented to illuminate an environment of the distal catheter section using electromagnetic radiation, and having an image recording unit (60), which is implemented to record an image of the electromagnetic radiation reflected from the environment of the distal catheter section and to conduct to the proximal end of the catheter (10), and an image display unit (62), which is connected to the proximal end of the catheter (10) and is implemented to display an image of the recorded electromagnetic radiation, the catheter (10) being implemented as steerable for insertion in body vessels, particularly blood vessels, and, in addition, for the purpose of displaying an image of the electromagnetic radiation reflected from the environment of the distal catheter section, having a wavelength for which blood has a high transparency, **characterized in that** the catheter (10) carries an expandable balloon on its distal catheter section, which is implemented as capable of dilating constricted body vessels.

2. The catheter according to Claim 1, **characterized in that** the catheter (10) is implemented to display an image recorded in a wavelength range from 600 to 650 nm.

3. The catheter according to Claim 2, **characterized in that** the illumination unit (40) is implemented to illuminate the environment of the distal catheter section using infrared light of a wavelength of 600 to 650 nm.

4. The catheter according to Claim 3, **characterized in that** the illumination unit (40) comprises an optical bandpass filter (48) for a frequency band from 600 to 650 nm.

5. The catheter according to one of Claims 1 through 4, **characterized in that** the illumination unit (40) comprises an illumination optical waveguide (50) from the proximal catheter end to the distal catheter end, which is implemented to conduct electromagnetic radiation used for illumination from the proximal catheter end to the distal catheter end.

6. The catheter according to one of the preceding claims, **characterized in that** the catheter (10) is implemented as an electrode line and is provided for this purpose on its distal catheter section with at least one electrode for delivering and/or recording electrical signals to or from body tissue adjoining the distal catheter section.

7. The catheter according to Claim 1, **characterized in that** the balloon is implemented as capable of inserting and expanding stents.

8. The catheter according to one of the preceding claims, **characterized in that** the catheter (10) comprises steering means (16), which may be actuated from the proximal end of the catheter (10), for the targeted deflection of the distal catheter end.

## Revendications

1. Cathéter endoscopique, conçu pour être introduit dans des cavités corporelles, comportant une section distale de cathéter et un dispositif d'éclairage (40) qui est conçu pour éclairer un environnement de la section distale du cathéter au moyen d'un rayonnement électromagnétique, une unité de prise d'images (60) qui est conçue pour prendre une image du rayonnement électromagnétique reflété par l'environnement de l'extrémité distale du cathéter et la conduire vers l'extrémité proximale du cathéter (10) ainsi qu'une unité de restitution d'images (62) qui est reliée à l'extrémité proximale du cathéter (10) et conçue pour restituer une image du rayonnement électromagnétique absorbé, le cathéter (10) est conçu pour être introduit de manière contrôlable dans des vaisseaux corporels, notamment des vaisseaux sanguins, ainsi que pour restituer une image du rayonnement électromagnétique reflété par l'environnement de la section distale du cathéter avec une longueur d'onde à laquelle le sang possède une transparence élevée, **caractérisé en ce que** le cathéter (10) supporte à son extrémité distale de cathéter un ballon expansible qui est apte à dilater des vaisseaux corporels rétrécis.

2. Cathéter selon la revendication 1, **caractérisé en ce que** le cathéter (1) est conçu pour restituer une image prise dans une plage de longueur d'onde allant de 600 à 650 nanomètres.

3. Cathéter selon la revendication 2, **caractérisé en ce que** le dispositif d'éclairage (40) est conçu pour éclairer l'environnement de l'extrémité distale du cathéter à la lumière infrarouge d'une longueur d'onde de 600 à 650 nanomètres.

4. Cathéter selon la revendication 3, **caractérisé en ce que** le dispositif d'éclairage (40) comprend un filtre passe-bande optique (48) pour une bande de fréquence de 600 à 650 nanomètres.

5. Cathéter selon une des revendications 1 à 4, **caractérisé en ce que** le dispositif d'éclairage (40) comprend une fibre optique d'éclairage (50) allant de l'extrémité proximale à l'extrémité distale du cathéter et qui est conçue pour diriger le rayonnement électromagnétique servant à l'éclairage de l'extrémité proximale à l'extrémité distale du cathéter.

6. Cathéter selon une des revendications précédentes, **caractérisé en ce que** le cathéter (10) est réalisé sous forme d'une ligne à électrode et est pourvu à cet effet au niveau de sa section distale de cathéter d'au moins une électrode pour émettre et/ou recevoir des signaux électriques au niveau ou en provenance du tissu corporel adjacent à la section distale du cathéter.

7. Cathéter selon la revendication 1, **caractérisé en ce que** le ballon est conçu pour être apte à être introduit dans des stents et les élargir.

8. Cathéter selon une des revendications précédentes, **caractérisé en ce que** le cathéter (10) comprend des moyens de contrôle (16) actionnables depuis l'extrémité distale du cathéter (10) pour la déviation ciblée de l'extrémité distale du cathéter.
